# EUROPEAN PATENT APPLICATION

(11) **EP 4 837 570 A1**
(43) Date of publication of application: **11.11.2026**
(21) Application number: 25174489.2
(22) Date of filing: 06.05.2025
(51) Int. Cl.: A61K 31/352, A61K 36/53, A61K 45/06, A61K 31/045, A61K 31/205, A61K 31/455, A61K 31/522, A61P 17/14

(54) **COMPOSITION COMPRISING CANNABINOIDS FOR USE IN THE PREVENTION AND TREATMENT OF HAIR LOSS**

(71) Applicant: UAB Sinela, 81107 Siauliu raj. (LT)
(72) Inventor: Voitechovicius, Andzejus, 14192 Avizieniai (LT)
(74) Representative: Metida

(57) **Abstract**

The invention provides a composition, composed of a synergistic mixture of natural extracts, cannabinoids, proteins, and essential nutrients. The composition is used for the prevention and/or treatment of alopecia and designed to stimulate hair follicle activity, prolong hair growth phase, inhibit the effects of DHT (dihydrotestosterone) on hair follicles, and improve scalp microcirculation.

The product is dermatologically tested, microbiologically safe, and free from harmful allergens. Clinical application and instrumental tests confirm its effectiveness in increasing hair density, reducing hair loss, improving scalp condition, and enhancing hair strength.

## Description

### TECHNICAL FIELD

The present invention relates to the field of products, particularly to a composition comprising cannabinoids for use in the preventing and/or treatment of alopecia and for non-therapeutic use in the stimulating hair growth and/or preventing or treating hair loss and improve scalp condition.

### BACKGROUND

Hair loss is a common concern affecting both men and women, often caused by factors such as aging, hormonal imbalances, stress, or environmental influences. Conventional hair loss treatments may cause side effects or offer limited effectiveness. Therefore, there is a need for a safe, effective, and natural cosmetic solution to prevent hair loss, promote hair growth, and improve overall scalp and hair health.

US2020022948 describes a method to reverse male pattern baldness by stimulating dormant hair follicles to re-enter the active growth phase (anagen). The approach combines an enzyme inhibitor to block dihydrotestosterone (DHT) synthesis, a flavonoid to increase certain prostaglandins, and a selected cannabinoid compound to restore normal hair follicle cycling. The difference is that cannabinoids are used to modulate DHT and prostaglandins, often alongside DHT blockers or flavonoids; present product has no DHT-modulating agents.

WO2021178871 describes topical formulations of cannabidiol, such as nanoparticulate cannabidiol, methods of making such compositions, and their use in treating autoimmune diseases and disorders, e.g., alopecia areata. This patent application focuses on nano-formulations for deeper follicle penetration; present product uses standard CBD without enhanced delivery systems.

US20230310305 discusses the use of cannabinoids such as tetrahydrocannabivarin (THCV) and cannabidivarin (CBDV) in promoting hair growth. These compounds act as CB1 antagonists, potentially stimulating hair follicle elongation and activating keratinocytes through endocannabinoid system receptors. The difference is that less common cannabinoids (varins) are used for acting as CB1 antagonists, promoting follicle elongation. These are not present in invented composition.

### BRIEF DESCRIPTION OF THE INVENTION

The invention provides a composition, composed of a synergistic blend of natural extracts, cannabinoids, proteins, and essential nutrients. The composition is designed to stimulate hair follicle activity, prolong hair growth phase, inhibit the effects of DHT (dihydrotestosterone) on hair follicles, and improve scalp microcirculation.

The product is dermatologically tested, microbiologically safe, and free from harmful allergens. Clinical application and instrumental tests confirm its effectiveness in increasing hair density, reducing hair loss, improving scalp condition, and enhancing hair strength.

A composition is intended for use in the prevention or treatment of androgenetic alopecia, comprising cannabinoids, selected from the group consisting of cannabidiol (CBD), cannabinol (CBN) and cannabigerol (CBG).

The composition comprises cannabidiol (CBD) amount in the range of 0.1% to 1.0% w/w, cannabinol (CBN) amount in the range of 0.1% to 1.0% w/w, and cannabigerol (CBG) amount the range of 0.1% to 1.0% w/w. The composition comprises cannabinoid total amount in the range of 0.3 - 3.0 w/w %, preferably 1.00 w//w %, and ethanol amount in the range of 50.0 - 75.0, preferably 55 w/w %.

In another embodiment the composition further comprises alcohol, water, Rosmarinus extract, glycerine, solubilizer, menthol, niacinamide, betaine, rice protein, caffeine, and ginger. The composition comprises caffeine amount in the range of 0.1% to 1.0% w/w, niacinamide amount in the range of 1.0% to 5.0% w/w, hydrolysed rice protein amount in the range of 0.1% to 1.0% w/w, Rosmarinus officinalis (rosemary) extract amount in the range of 5.0% to 10.0% w/w, Zingiber officinale (ginger) root extract amount in the range of 0.1% to 1.0% w/w, L-menthol amount in the range of 1.0% to 5.0% w/w, betaine amount in the range of 0.1% to 1.0% w/w, vegetable glycerine amount in the range of 1.0% to 5.0% w/w, alcohol amount in the range of 50.0% to 75.0% w/w, distilled water amount in the range of 10.0% to 25.0% w/w.

The composition further comprises preservatives, solubilizers and essential oil components, wherein the solubilizer amount is in the range of 1.0% to 5.0% w/w, the preservative amount is in the range of 0.1% to 1.0% w/w. Essential oil-derived components, natural allergens from rosemary present in trace amounts, including Linalool, D-Limonene, Pinene, Terpinene, and 3-Carene (no more than 0.1% w/w).

The alcohol is selected from ethanol and methanol.

The preservatives are selected from phenoxyethanol, ethylhexylglycerin, potassium sorbate, sodium benzoate.

The solubilizer is Polysorbate 20.

The composition is prepared in liquid form.

The composition is administered by topical route. 5 to 10 drops of the composition are applied to clean, dry scalp 2 to 3 times a week.

In another embodiment the composition comprising cannabinoids, selected from the group consisting of cannabidiol (CBD, cannabinol (CBD) and cannabigerol in intended for non-therapeutic use in the stimulating hair growth and/or preventing or treating hair loss and improve scalp condition.

The cosmetic composition further comprises alcohol, water, Rosmarinus extract, glycerine, solubilizer, menthol, niacinamide, betaine, rice protein, caffeine, ginger, preservatives, solubilizers and cosmetic allergens.

### BRIEF DESCRIPTION OF DRAWINGS

Fig. 1 illustrates increasing of hair density before application of the composition and after 28 days of regular application of the composition.
Fig. 2 illustrates hair growing results before product application and after 28 days of regular product application.
Fig. 3 illustrates scalp condition before product application and after 28 days of regular product application.
Fig. 4 shows androgenic alopecia before product application and after 28 days of regular product application.

### DETAILED DESCRIPTION OF INVENTION

This invention provides the composition for use in preventing and treatment of androgenetic alopecia and for non-therapeutic use in preventing of hair loss, enhancing scalp and hair health and promoting hair growth comprising cannabinoids. The cannabinoids are selected from Cannabidiol (CBD), Cannabinol (CBN), and Cannabigerol (CBG).

Cannabidiol (CBD) interacts with the endocannabinoid system (ECS), particularly the CB1 receptors in hair follicles. By modulating these receptors, CBD may prolong the growth phase of the hair cycle, promoting hair growth. A study involving individuals with androgenetic alopecia observed a 93.5% average increase in hair count after six months of daily topical CBD application. CBD's anti-inflammatory effects can alleviate scalp conditions like psoriasis and seborrheic dermatitis, reducing itching and irritation. CBD may help balance oil production on the scalp, addressing issues related to both dryness and excessive oiliness.

Cannabinol (CBN) interacts with the ECS, its structural similarity to CBD suggests it may offer comparable benefits, such as supporting hair growth and scalp health. CBN is known for its mild sedative and antioxidant properties, which might help reduce stress-related hair loss by promoting relaxation.

Cannabigerol (CBG) binds to both CB1 and CB2 receptors in the ECS, potentially influencing hair follicle function and promoting a healthy scalp environment. CBG exhibits anti-inflammatory properties, which can help soothe the scalp and reduce conditions that may lead to hair loss. CBG may support hair growth by interacting with ECS receptors in the skin and hair follicles.

Synergistic effects of the combination of CBD, CBN, and CBG in the composition aims to harness their collective benefits. Broad-spectrum cannabinoids promote scalp health and hair follicle stimulation. By modulating ECS receptors, these cannabinoids may collectively promote the anagen phase of hair growth. Their anti-inflammatory and antioxidant properties can soothe the scalp, reducing conditions that hinder hair growth. CBN's sedative effects may help mitigate stress-related hair loss.

The composition further comprises alcohol, water, Rosmarinus extract, glycerine, solubilizer, menthol, niacinamide, betaine, rice protein, caffeine, ginger, and preservatives.

The following particular ingredients, each of which has required characteristic for the final product, are used in the composition:
Water is distilled water.
Alcohol is selected from ethanol or methanol and acts as carrier and preservative. Rosmarinus officinalis (Rosemary) extract: Enhances microcirculation, strengthens hair roots, and has antioxidant properties. Rosemary extract is prepared from dried rosemary leaves which are mixed with the ethanol 70% in a ratio 1:5 (e.g., 100 g herb + 500 mL solvent). After maceration of leaved the extract is filtrated and concentrated.
Zingiber officinale (Ginger) root extract: Stimulates hair follicles and improves blood flow. Hydrolysed Rice Protein: Strengthens hair fibers and supports follicle vitality.
Caffeine: Inhibits 5α-reductase, reducing DHT levels and promoting hair growth. Niacinamide (Vitamin B3): Improves scalp barrier function and hydration.
L-Menthol: Provides a cooling sensation, soothes the scalp, and enhances absorption. Glycerine and Betaine: Act as humectants to maintain scalp hydration.
Preservatives are selected from Phenoxyethanol, Ethylhexylglycerin, Potassium Sorbate, and Sodium Benzoate and ensures product stability and safety.
Essential oil components are selected from Linalool, D-limonene, pinene, terpinene, 3-carene. These are naturally present in rosemary or can be added optionally separately for fragrance/allergen declaration.
The composition is alcohol-based (using alcohol derived from rosemary extract), achieving a natural preservation effect and contributing to scalp freshness. The prepared composition is transparent liquid which pH is 5.5 and density at 25°C is 0.92g/ml.

The amounts of the ingredients of the composition are provided below in Table 1.

**Table 1**

| Ingredient | Amount % w/w |
|---|---|
| Alcohol | 50.0 - 75.0 |
| Distilled water | 10.0 - 25.0 |
| Rosemary officinalis extract | 5.0 - 10.0 |
| Vegetable glycerine | 1.0 - 5.0 |
| Solubiliser | 1.0 - 5.0 |
| L-menthol | 1.0 - 5.0 |
| Niacinamide | 1.0 - 5.0 |
| Betaine | 0.1 - 1.0 |
| Hydrolysed rice protein | 0.1 - 1.0 |
| Caffeine | 0.1 - 1.0 |
| Ginger root extract | 0.1 - 1.0 |
| Cannabidiol | 0.1 - 1.0 |
| Cannabinol | 0.1 - 1.0 |
| Cannabigerol | 0.1 - 1.0 |
| Preservatives | 0.1 - 1.0 |
| Cosmetic allergens | ≤ 0.1 |

### Example of anti-hair loss serum composition (% w/w):

Ethanol - 55.00
Distilled water - 24.00
Rosmary officinalis extract - 6.00
Vegetable glycerine - 3.00
Polysorbate 20 - 2.00
L-menthol - 2.50
Niacinamide (vitamin B3) - 2.50
Betaine - 0.80
Hydrolysed rice protein - 0.80
Caffeine - 0.80
Ginger root extract - 0.80
Cannabidiol (CBD) - 0.50
Cannabinol (CBN) - 0.20
Cannabigerol (CBG) - 0.30
Phenoxyethanol - 0.70
Linalool, D-limonene, Pinene, Terpinene, 3-carene - 0.10.

The provided composition ensures broad-spectrum protection without parabens.

The synergistic effect of ingredients (caffeine, L-menthol, ginger and niacinamide) improves oxygen and nutrient delivery to follicles, prolonging the growth phase and reviving dormant follicles. The components such as CBD, CBG, CBN, Rosemary extract, betaine and niacinamide suppress chronic micro-inflammation around the hair bulb - an early hallmark of alopecia - and protect cells from free radical damage. Hydrolysed rice protein and caffeine support the physical structure of growing hair, making it thicker and less prone to breakage. Alcohol and Polysorbate 20 improve solubility and delivery of cannabinoids and plant extracts and preservatives provide full-spectrum preservation, ensuring product safety. The solvent and surfactant system ensures effective penetration of actives, particularly cannabinoids, while the preservatives maintain product stability without disrupting scalp microbiota.

The composition works synergistically by enhancing follicular microcirculation, reducing local inflammation and oxidative stress, restoring scalp hydration and barrier function; strengthening the structural integrity of hair and optimizing delivery and activity of cannabinoids and bioactives. This combination results in longer anagen phases, less hair shedding, and thicker, healthier hair regrowth, supported by both plant-based and cannabinoid ingredients.

The method of production of composition comprises mixing of two phases A and B, addition of essential oil ingredients with solubilizer to the mixture and final step by adding prepared cannabinoid mixture.

Aqueous Phase A: distilled water is heated to 55-60°C in a mixing vessel. The caffeine is added slowly under stirring and continue mixing until the caffeine is fully dissolved. Then glycerine is added and mixed, after niacinamide is added and mixed until dissolved. The remaining water-soluble ingredients - hydrolysed rice protein, betaine, ginger root extract and preservatives - are added and stirred until uniform mixture.

Alcoholic Phase B: In a separate vessel alcohol and menthol are mixed and stirred until menthol is completely dissolved. This typically occurs at room temperature or slightly warmed (up to 30°C).

When phases A and B are prepared, Phase B is added into Phase A under continuous mixing. The temperature is maintained at room temperature (~25°C) during mixing.

The solubiliser is added to the prepared mixture and mixed for 5-10 minutes, the Rosemary extract is added under continuous stirring and, optionally, the essential oil components are added. The mixture is stirred for at least 15-20 minutes to ensure uniform dispersion.

In the final step, Cannabidiol (CBD), Cannabinol (CBN) and Cannabigerol (CBG) are dissolved in a small portion of ethanol. The cannabinoid mixture is added slowly into the main mixture under gentle agitation and mixing for at least 20 minutes to ensure full dispersion and stability.

The prepared mixture is clear or slightly opalescent liquid, final pH is about 5.5, density about 0.92 g/ml at 25°C comprising cannabinoid total amount in the range of 0.3 - 3.0 % w/w, preferably 1.00 % w/w and ethanol content in the range of 50.0 - 75.0, preferably 55 % w/w.

The composition is administered by topical use. 5 to 10 drops of the composition are applied to clean, dry scalp, massaging gently to promote absorption. Leave on for 5-10 minutes without rinsing. For best results, use 2 to 3 times a week.

The composition is intended for use in the treatment or prevention of androgenetic alopecia. In addition, the composition in intended for use as cosmetic composition in non-therapeutic use in suppressing, improving or preventing hair loss and/or suppressing or improving hair growing.

Based on available information from the ingredient specification, the ingredients used are assessed as microbiologically harmless.

The microbiological specifications of broad cannabinoid spectrum are provided in Table 2.

**Table 2**

| **CHARACTERISTICS** | **METHOD (PNT)** | **SPECIFICATION** | **RESULTS** |
|---|---|---|---|
| AEROBIC COUNT | PNT-015 | <1000 CFU/g or ml | <10 CFU/g |
| COUNTING MOLD AND YEASTS | PNT-015 | <1000 CFU/g or ml | <10 CFU/g |
| Staphylococcus aureus | PNT-015 | ABSENCE | Absence |
| Pseudomonas aeruginosa | PNT-015 | ABSENCE | Absence |
| Candida albicans | PNT-015 | ABSENCE | Absence |
| Escherichia coli | PNT-015 | ABSENCE | Absence |

The synergistic effects of the composition are provided in the tests below.

### Dermatological open skin test under the supervision of a dermatologist

The aim of the study was to assess the irritating and sensitizing properties (skin tolerance) of the product on a healthy adult skin by allowing the product to have longer contact with the skin. Exclusion/confirmation of the irritating and sensitizing potential of the product was done by a single application of the product to the human skin and evaluation of skin irritation after established time periods.

Trials were read 15 minutes, 2h and 5h after product application:
P0 - test start, product application
P1 - reading after 15 min
P2 - reading after 2h
P3 - reading after 5h
Place of product application: forearm.
Concentration of tested product: pure 100%.
Number of volunteers: 10 volunteers with normal skin.
Study interpretation: A positive reaction (erythema) confirms the allergenic properties of the product, while a negative result (no erythema) confirms the absence of allergenic properties. The final result is the average value of the reading in all test subjects.

The Table 3 shows the scale for evaluating test results. Interpretation of the results follows the recommendations of the International Contact Eczema Research Group (ICDRG):

**Table 3**

| **Symbol** | **Evaluation** | **Interpretation** |
|---|---|---|
| - | Negative reaction | No reaction |
| ? | Doubtful reaction | Subtle erythema, palpation undetectable erythematous spot |
| + | Low reaction | Palpationally palpable erythematous focus suggestive of mediocre edema/infiltration, with or without papules, without vesicles |
| ++ | Strong reaction | Vesicles present, increased edema, infiltration, papules |
| +++ | Very strong reaction | Blisters formed by confluence of blisters or erosions, ulcerations |
| IR | Irritant reaction | Shiny skin, dry, pustules, erythema, tendency to fade out |

Results of test are presented below in Table 4:

**Table 4**

| Volunteer | Code | Age | Gender | Place of skin application | Result P1 | Result P2 | Result P3 |
|---|---|---|---|---|---|---|---|
| 1. | KP | 32 | F | forearm | (-) | (-) | (-) |
| 2. | YM | 32 | F | forearm | (-) | (-) | (-) |
| 3. | GR | 38 | M | forearm | (-) | (-) | (-) |
| 4. | EF | 65 | F | forearm | (-) | (-) | (-) |
| 5. | KJ | 23 | F | forearm | (-) | (-) | (-) |
| 6. | NP | 25 | F | forearm | (-) | (-) | (-) |
| 7. | PP | 35 | F | forearm | (-) | (-) | (-) |
| 8. | AH | 30 | F | forearm | (-) | (-) | (-) |
| 9. | AS | 50 | F | forearm | (-) | (-) | (-) |
| 10. | AS | 26 | F | forearm | (-) | (-) | (-) |
| SUMMARY | NUMBER OF PEOPLE STARTING THE STUDY: 10 | THE AVERAGE: 36 | F/M | forearm - 100% | Result (-) - 100% | Result (-) - 100% | Result (-) - 100% |
| | | | F 90% | | | | |
| | NUMBER OF PEOPLE FINISHING THE TEST: 10 | | | | | | |
| | | MIN: 23 | M 10% | | | | |
| | | MAX: 65 | | | | | |

| | | | | | | | |
|---|---|---|---|---|---|---|---|
| F-female, M-male P1 - reading after 15 min P2 - reading after 2h P3 - reading after 5h | | | | | | | |

Dermatological testing for the product SS Hair loss serum was performed with an open dermatological test on a group of 10 volunteers. In 100% of those who underwent dermatological testing, there were no positive reactions or irritant reactions. The test confirmed that the product is NON-irritating to the skin and confirmed the declaration "Dermatologically tested product".

### Instrumental test of cosmetic product

The aim of the test -confirmation / exclusion of the product properties declared by the manufacturer and its operation on the basis of an instrumental test:
- Hair density measurement T0 before study, T1 after 28 days of regular product application - Aramo ASW 300 x60
- Scalp and hair condition assessment T0 before study, T1 after 28 days of regular product application - Aramo ASW 300 x60
- Androgenetic alopecia assessment T0 before study, T1 after 28 of regular product application - Canon 250D
- Brush test T0 before study, T1 after 28 days of regular product application Research with the participation of a properly selected group of volunteers - characteristics of the research group in accordance with the approved research program. Selection of volunteers based on the inclusion and exclusion criteria specified in the consent form for participation in the study (Table 5).

**Table 5**

| | | |
|---|---|---|
| CRITERIA | Number of volunteers in the study: | 10 |
| | Sex: | female, male |
| | Age: | 18 + |
| | Phototype: | I-IV |
| | Scalp type: | all type |
| | Skin sensitivity: | sensitive /nonsensitive |

Measurements were carried out using Aramo ASW300 x60 and its dedicated software. Measurements were done on the same part of the scalp and hair before and after 28 days of regular product usage.

Photos were taken by Canon 250D camera equipped with EFS 18 - 55 mm lens. Camera worked on manual settings with aperture F16, exposure time 125s, ISO 100 and focal length 55 mm. Camera was also equipped with Yongnuo YN24EX external lamp, that worked in ETTL mode and with lamp exposure correction -1/3. Two photos at different angles were taken before
application of the product and after 28 days of regular product usage. All before and after photos were taken in the same room, with the same, constant lighting. Volunteers and laboratory team were equipped with special grey capes to avoid colour interference on photos.

The brush test was conducted using a comb with a length of 12.5 cm. A specific section of hair was separated from the rest, and then it was combed 5 times, starting from the crown, continuing through the occipital area, and ending at the nape. After performing 5 combing strokes, the hairs that fell out, as well as those left on the comb, were counted. All measurements were carried outcondit in a room with a temperature of 22±3° C and a humidity of 40-60%.

The effectiveness of the product is confirmed by positive results in at least 50% of the probands participating in the study.

Hair density measurement is provided in Table 6.

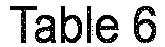

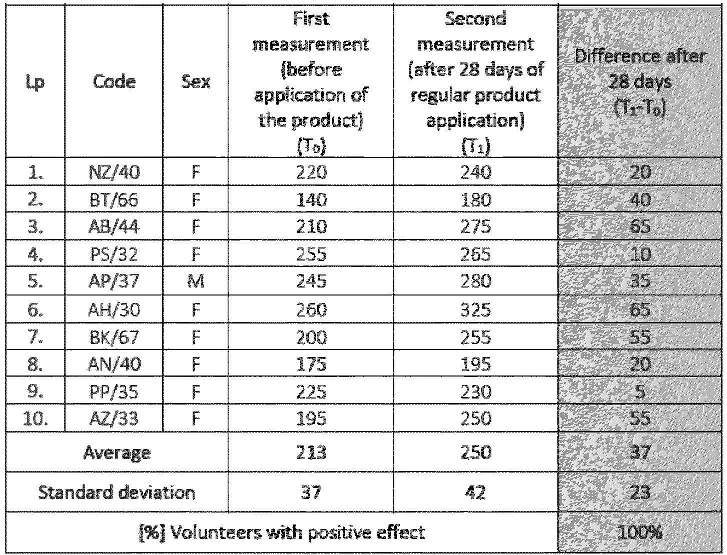

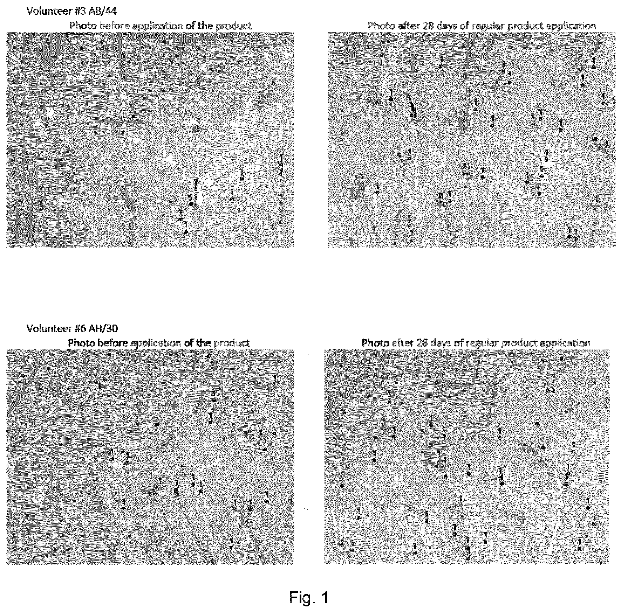

Higher result on second (T1) measurement in comparison to the first (T0) one means increase in hair density. Fig. 1 shows hair density results before application of the composition and after 28 days of regular application of the composition.

*The results shown in the table are rounded to 0 decimal places.

Scalp and hair condition assessment is provided in Table 7.

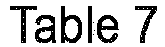

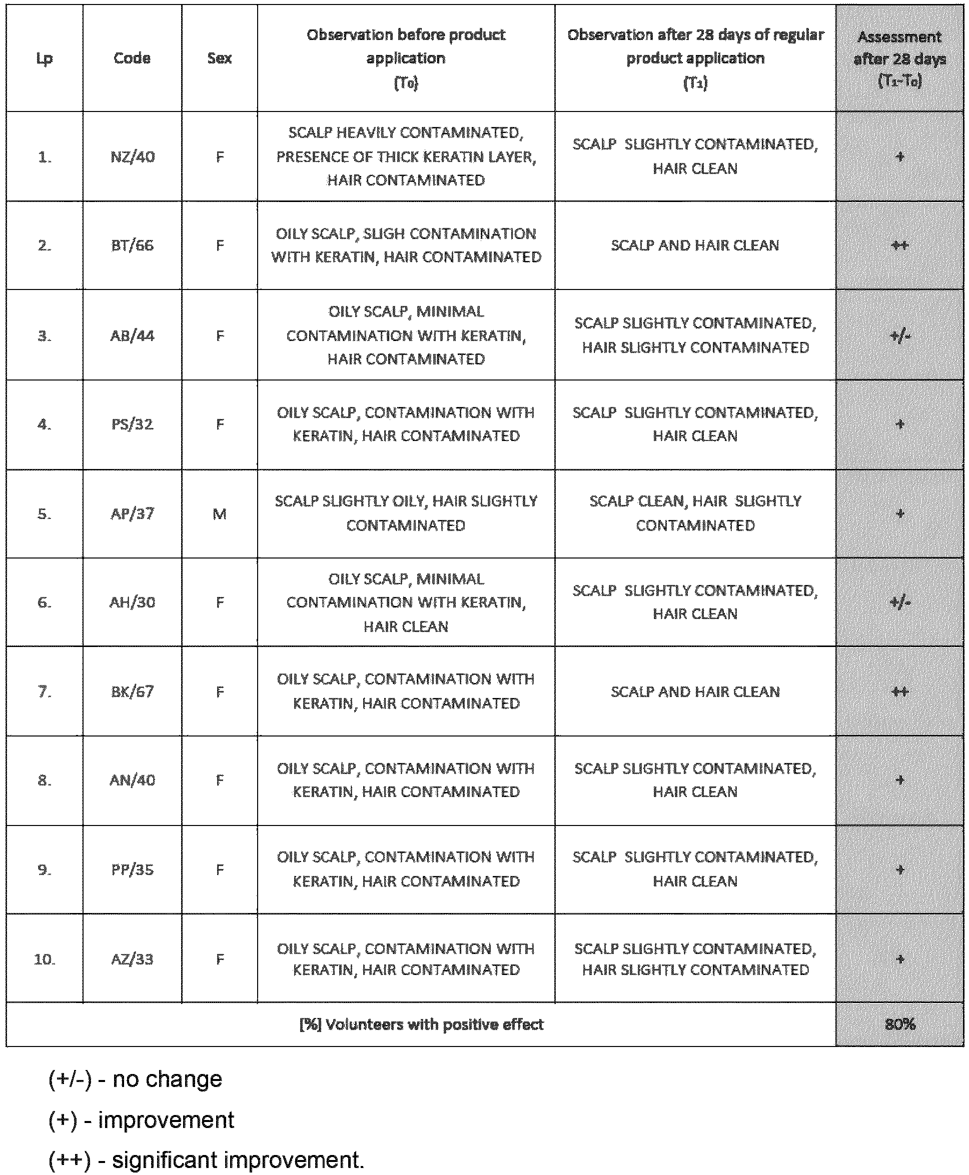

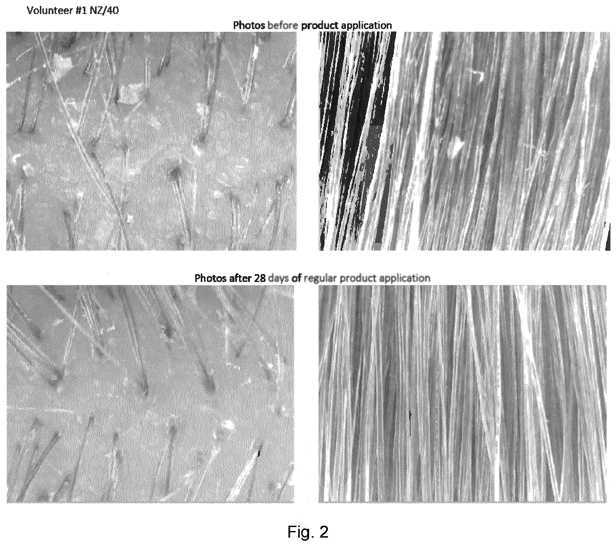

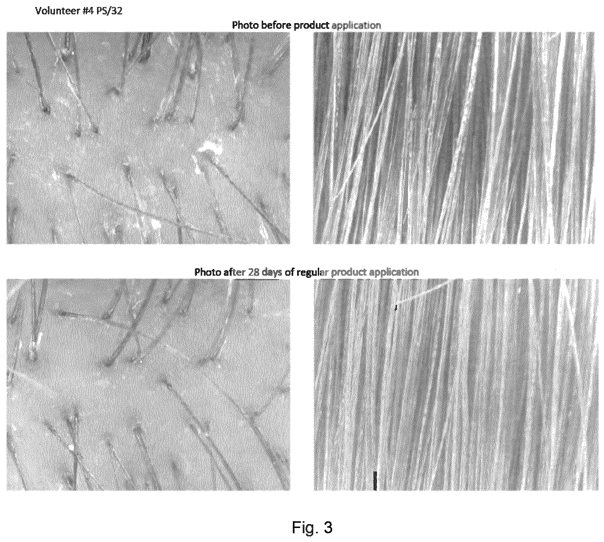

Figure 2 shows results before product application and after 28 days of regular product application. Fig. 3 illustrates scalp condition before product application and after 28 days of regular product application.

### Androgenetic alopecia assessment

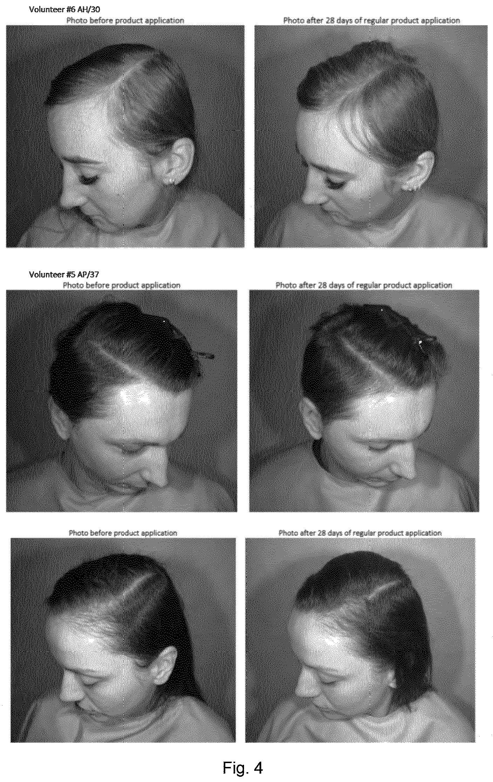

Fig. 4 shows androgenic alopecia before product application and after 28 days of regular product application.

Brush test is provided in Table 8.

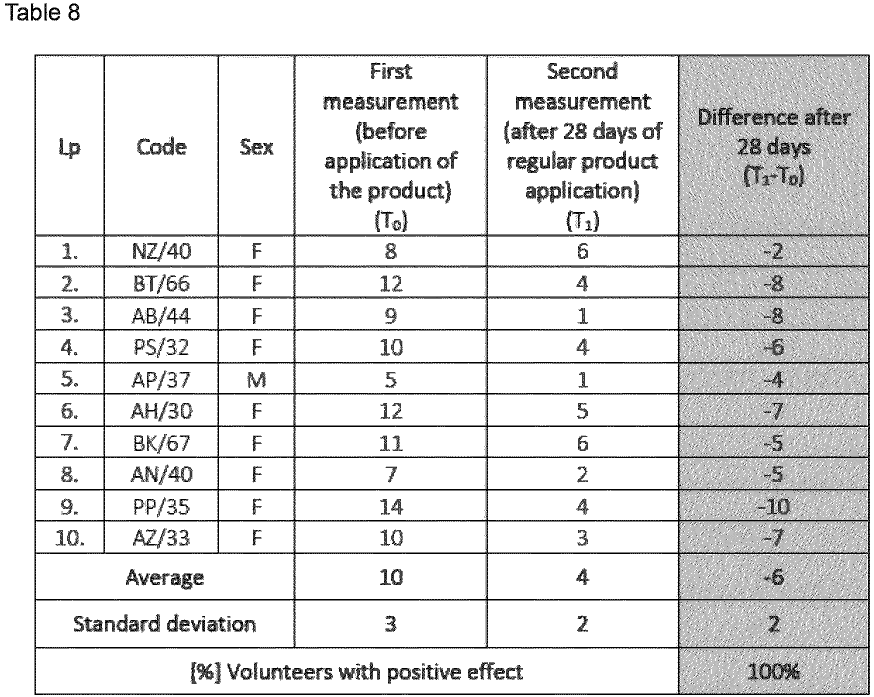

Assumption: Lower result on second (T1) measurement in comparison to the first (T0) one means a reduction of hair loss.

On the basis of the instrumental analyzes, it was found that the tested product:
- Increases hair density by an average of 17% after 28 days of regular application, hence confirming the claim, that the product makes hair stronger;
- Improves the condition of hair and scalp after 28 days of regular application, hence confirming the claim, that the product improves scalp condition;
- Visibly reduces degree of androgenic alopecia after 28 days of regular application, hence confirming the claim, that the product promotes the growth of thicker hair;
- Reduces hair loss by an average of 63% after 28 days of regular application, hence confirming the claim, that the product reduces hair loss.

### Application and use test of a cosmetic product

The aim of the test is:
- Assessment of skin tolerance as a result of long-term, repeated application when the product is used as intended and in accordance with the declared method of use.
- Confirmation/exclusion for the product performance declared by the manufacturer.

### Evaluation of features and product properties:

Fragrance of the product
Colour of the product
Efficiency of the product
Texture of the product

### Overall product assessment:

Evaluation of the product compared to other products of this type
Willingness to buy this product
Willingness to recommend this product

### Overall assessment of product tolerance

Negative feelings during use
Marketing assessment:
   - the product improves the condition of the scalp
   - the product reduces hair loss
   - the product strengthens the hair
   - the product increases hair thickness
Test duration:
   28 days ± 4 days

The use test was performed on a group of healthy adult volunteers at home during regular repeated application. In accordance with the application research program approved by the client before the start of the study. The scope of questions in the final questionnaire for volunteers, the characteristics of the research panel, the duration of the study and the number of study participants were consistent with the client's requirements.

The volunteers were healthy, with negative history of allergy. The selection of the group included the criteria of inclusion and exclusion. None of the volunteers reported documented oversensitivity or a history of adverse reactions to individual ingredients of the tested product. All volunteers fulfilled the requirements of inclusion for tests. The examination was performed according to an internal procedure. Selection of probands based on inclusion and exclusion criteria. The test was performer on healthy skin without irritation. Volunteers were advised not to use any antihistamines and pharmacological drugs that could affect the test result. A study involving a properly selected group of volunteers. The test was performed according to the internal procedure. Table 9 shows characteristic of the research panel.

**Table 9**

| | | |
|---|---|---|
| CHARACTERISTICS OF THE RESEARCH PANEL | Number of volunteers: | 10 |
| | Sex: | Women and men |
| | Age: | 18+ |
| | Skin phototype: | I-IV |
| | Place of application: | Scalp |
| | Skin type: | All skin type |
| | Skin sensitivity: | Sensitive/non-sensitive |
| | Cosmetics study program: | PB/317/12/2024/V1 |

Description of the panel of volunteers participating in the study is provided in Table 10. Skin phototypes are indicated according to the Fitzpatrick classification.

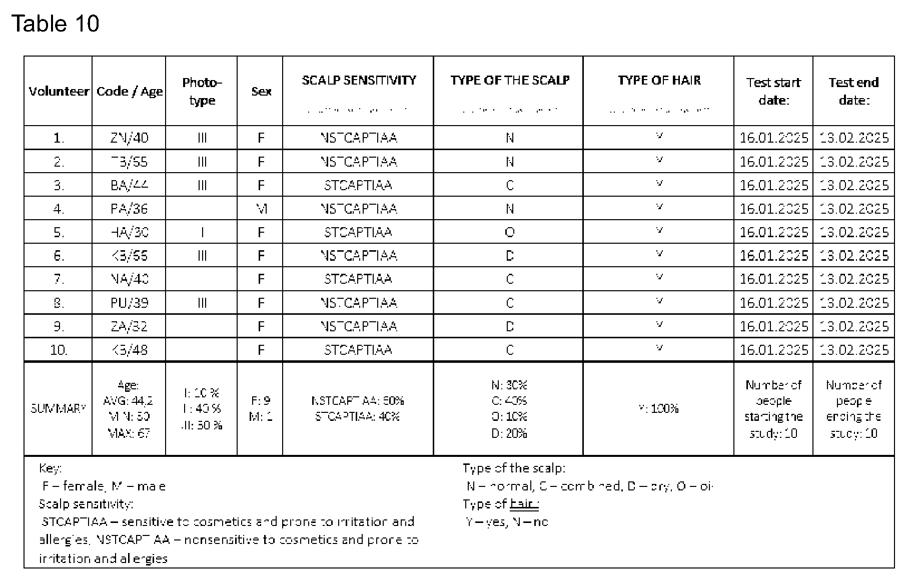

The test product was well tolerated by the skin during the application period. During the application, no negative symptoms indicating intolerance to the product or hypersensitivity to any of the ingredients of the preparation have been observed and have not been found.

### Evaluation of features and product properties

**Table 11**

| **I. EVALUATION OF FEATURES AND PRODUCT PROPERTIES** | | | | | |
|---|---|---|---|---|---|
| | | Totally agree | Agree | Not agree | Not agree at all |
| 1 | Smell of the product | 40,00% | 50,00% | 10,00% | 0,00% |
| | | Totally agree | Agree | Not agree | Not agree at all |
| 2 | colour of the product | 50,00% | 50,00% | 0,00% | 0,00% |
| | | Totally agree | Agree | Not agree | Not agree at all |
| 3 | Efficiency of the product | 50,00% | 50,00% | 0,00% | 0,00% |
| | | Totally agree | Agree | Not agree | Not agree at all |
| 4 | Efficiency of spreading the product | 50,00% | 50,00% | 0,00% | 0,00% |

### Overall product assessment

**Table 12**

| **II. OVERALL PRODUCT ASSESSMENT** | | | | | |
|---|---|---|---|---|---|
| | | Definitely better | Better | Comparable | Definitely worse |
| 5 | Evaluation of the product compared to other products of this type | 30,00% | 50,00% | 20,00% | 0,00% |
| | | I would definitely buy it | I would buy it | I don't know if i would buy it | I would not definitely buy it |
| 6 | Would you like to buy this product? | 30,00% | 50,00% | 20,00% | 0,00% |
| | | Totally agree | Agree | Not agree | Not agree at all |
| 7 | Would you like to recommend this product? | 50,00% | 50,00% | 0,00% | 0,00% |

### Overall assessment of product tolerance

**Table 13**

| **III. OVERALL ASSESSMENT OF PRODUCT TOLERANCE** | | | | | |
|---|---|---|---|---|---|
| | | No | Yes | - | - |
| 8 | Has the product caused any negative feelings during use? | 100,00% | 0,00% | - | - |

### Detailed description of volunteer's panel

**Table 14**

| **IV. DETAILED DESCRIPTION OF VOLUNTEER'S PANEL** | | | | | |
|---|---|---|---|---|---|
| | | Sensitive to cosmetics and prone to Irritation and allergies | Nonsensitive to cosmetics and prone to irritation and allergies | - | - |
| 9 | SCALP SENSITIVITY | 40,00% | 60,00% | - | - |
| | | Normal | Combined | Dry | Oil |
| 10 | TYPE OF THE SCALP | 30,00% | 40,00% | 20,00% | 10,00% |
| | | Yes | No | - | - |
| 11 | TYPE OF HAIR | 100,00% | 0,00% | - | - |

### Marketing assessment

**Table 15**

| **V. MARKETING ASSESSMENT** | | | | | |
|---|---|---|---|---|---|
| | | Totally agree | Agree | Not agree | Not agree at all |
| 12 | Do you think that the product improves the condition of the scalp? | 30,00% | 60,00% | 10,00% | 0,00% |
| | | Totally agree | Agree | Not agree | Not agree at all |
| 13 | Do you think that the product reduces hair loss? | 20,00% | 70,00% | 10,00% | 0,00% |
| | | Totally agree | Agree | Not agree | Not agree at all |
| 14 | Do you think that the product strengthens the hair? | 20,00% | 70,00% | 10,00% | 0,00% |
| | | Totally agree | Agree | Not agree | Not agree at all |
| 15 | Do you think that the product increases hair thickness? | 20,00% | 50,00% | 30,00% | 0,00% |

### Overall product assessment

The study confirmed the following claims:
- Evaluation of the product compared to other products of this type (100,00% positive answers)
- Willingness to buy this product (80,00% positive answers)
- Willingness to recommend this product (100,00% positive answers).

### Overall assessment of product tolerance

The study did not confirm the following:
- Negative feelings during use (0,00% positive answers)

### Marketing assessment

The study confirms the following:
- the product improves the condition of the scalp (90,00% positive answers)
- the product reduces hair loss (90,00% positive answers)
- the product strengthens the hair (90,00% positive answers)
- product increases hair thickness (70,00% positive answers)

The composition provides a scientifically validated solution for use is the treatment and prevention of androgenetic alopecia, for the stimulating hair growth and/or preventing or treating hair loss and improve scalp condition. It is natural-based composition, supported by clinical studies, ensures safety, efficacy, and a positive user experience. This combination of cannabinoids, botanical extracts, proteins, and caffeine delivers synergistic technical effects that meet consumer demand for effective, safe, and innovative hair care solutions.

## Claims

1. A topical composition, comprising cannabinoids, selected from the group consisting of cannabidiol (CBD), cannabinol (CBN) and cannabigerol (CBG), for use in the treatment or prevention of androgenetic alopecia.

2. The composition for use according to claim 1, wherein composition comprises cannabidiol (CBD) amount in the range of 0.1% to 1.0% w/w, cannabinol (CBN) amount in the range of 0.1% to 1.0% w/w, and cannabigerol (CBG) amount in the range of 0.1% to 1.0% w/w.

3. The composition for use according to claims 1 and 2, wherein the composition further comprises alcohol, water, Rosmarinus extract, glycerine, solubilizer, menthol, niacinamide, betaine, rice protein, caffeine, and ginger.

4. The composition for use according to any of claims 1-3, wherein the composition comprises caffeine amount in the range of 0.1% to 1.0% w/w, niacinamide amount in the range of 1.0% to 5.0% w/w, hydrolysed rice protein amount in the range of 0.1% to 1.0% w/w, Rosmarinus officinalis (rosemary) extract amount in the range of 5.0% to 10.0% w/w, Zingiber officinale (ginger) root extract amount in the range of 0.1% to 1.0% w/w, L-menthol amount in the range of 1.0% to 5.0% w/w, betaine amount in the range of 0.1% to 1.0% w/w, vegetable glycerine amount in the range of 1.0% to 5.0% w/w, alcohol amount in the range of 50.0% to 75.0% w/w, and distilled water amount in the range of 10.0% to 25.0% w/w.

5. The composition for use according to any of claims 1-4, wherein the composition further comprises preservatives, solubilizers and cosmetic allergens.

6. The composition for use according to any of claims 1-5, wherein the composition comprises the solubilizer amount in the range of 1.0% to 5.0% w/w and the preservatives amount in the range of 0.1% to 1.0% w/w, and the essential oil components amount no more than 0.1% w/w.

7. The composition for use according to claim 6, wherein preservatives are selected from phenoxyethanol, ethylhexylglycerin, potassium sorbate, sodium benzoate.

8. The composition for use according to claim 6, wherein solubilizer is Polysorbate 20.

9. The composition for use according to claim 6, wherein the essential oil components are selected from Linalool, D-limonene, Pinene, Terpinene, 3-carene.

10. The composition for use according to any of claims 1-9, wherein the composition is in liquid form.

11. The composition for use according to any of claims 1-10, wherein the composition is in a form of topic administration.

12. The composition for use according to any of claims 1-11, wherein 5 to 10 drops of the composition are applied to clean, dry scalp 2 to 3 times a week.

13. Non-therapeutic use of a composition comprising cannabinoids, selected from the group consisting of cannabidiol (CBD, cannabinol (CBD) and cannabigerol for the stimulating hair growth and/or preventing or treating hair loss and improve scalp condition in an individual.

14. Non-therapeutic use of a composition according to claim 13, wherein the composition further comprises alcohol, water, Rosmarinus extract, glycerine, solubilizer, menthol, niacinamide, betaine, rice protein, caffeine, ginger, preservatives, solubilizers and cosmetic allergens.

15. Non-therapeutic use of a composition according to claims 13 and 14, wherein the composition is in a form for topic administration.
